# EUROPEAN PATENT APPLICATION

(11) **EP 3 659 567 A1**
(43) Date of publication of application: **03.06.2020**
(21) Application number: 19210181.4
(22) Date of filing: 19.11.2019
(51) Int. Cl.: A61G 7/10, B66C 1/10, G09F 3/00

(54) **RAIL-MOUNTED LIFT SYSTEMS WITH LOCATION TRACKING, SUBJECT TRACKING, AND USER TRACKING CAPABILITIES**

(30) Priority: 28.11.2018 US 201862772168 P
(71) Applicant: Liko Research & Development AB, 975 92 Luleå (SE)
(72) Inventor: LEDWITH, James, Batesville, IN 47006 (US); STANCATO, Melissa, Batesville, IN 47006 (US); RUD, Svetlana, Batesville, IN 47006 (US); BERKEBILE, Jacqueline, Batesville, IN 47006 (US); AGGARWAL, Saumil, Batesville, IN 47006 (US); DAVIDSON, Frederick Collin, Batesville, IN 47006 (US); AJAYI, Abidemi, Batesville, IN 47006 (US); TARI, Joseph, Batesville, IN 47006 (US); STRASSIE, Derek, Batesville, IN 47006 (US)
(74) Representative: Findlay, Alice Rosemary

(57) **Abstract**

Rail-mounted lift systems, systems comprising the same, and kits of parts for retrofitting rail-mounted lift systems are disclosed. A lift system includes a rail having a carriage support channel formed therein, a carriage slidably disposed in the carriage support channel for relative movement to the rail, a lift unit coupled to the carriage, the lift unit having a motor that extends and retracts a lifting strap, and a locating system having a first component and a second component separate from the first component. One of the first and second components is a transmitter providing a code that corresponds to a predetermined location and the other is a receiver detecting the code when the first and second components are moved in proximity to one another due to movement of the carriage along the rail.

## Description

The present specification generally relates to rail-mounted lift systems and, more specifically, to rail-mounted lift systems with sensing components that can be used to determine a location of the lift system, associate a user with the lift system, and/or associate a subject with the lift system.

Overhead lifting devices, such as subject lifts used in the health care industry, may generally include a lift unit with an actuator, such as an electric motor or similar actuator, coupled to a mechanical lifting arm or cable lift system, such as a lifting strap. The actuator facilitates actuation of the mechanical lifting arm or cable lift system, thereby raising and/or lowering a load attached to the lifting arm or cable lift system. The lift unit may be coupled to a rail system with a carriage which facilitates positioning the lift unit with respect to the rail. Positioning the unit along the rail system may be accomplished manually or, in the alternative, with a motor mechanically coupled to the carriage and operable to traverse the carriage and lift unit over the span of the rail system.

The rails may be positioned at various locations throughout a space, such as in a hospital. For example, the rails may be positioned such that the overhead lifting device can be transported via the rails throughout a hospital, such as into rooms, through corridors, into therapy rooms, into operating rooms, and/or the like. Accordingly, it becomes necessary to track the location of the overhead lifting device. In addition, since multiple subjects may be located in each of these spaces, it becomes necessary to discern which subject is being lifted by a particular lifting device, as well as whether the appropriate lift equipment is being used (e.g., components and/or accessories) for a particular subject.

In one aspect, a rail-mounted lift system includes a rail having a carriage support channel formed in the rail, a carriage slidably disposed in the carriage support channel of the rail for relative movement to the rail, a lift unit coupled to the carriage, the lift unit having a motor that extends and retracts a lifting strap, and a locating system having a first component and a second component that is separate from the first component. One of the first component and the second component is a transmitter providing a code that corresponds to a predetermined location and another one of the first component and the second component is a receiver detecting the code when the first component and the second component are moved in proximity to one another due to movement of the carriage along the rail.

In another aspect, a system includes a server computing device and a rail-mounted lift system. The rail-mounted lift system includes a rail having a carriage support channel formed in the rail, at least one assembly including a carriage slidably disposed in the carriage support channel of the rail for relative movement to the rail, and a locating system having a first component and a second component that is separate from the first component. The at least one assembly is communicatively coupled to the server computing device. One of the first component and the second component is a transmitter providing a code that corresponds to a predetermined location and another one of the first component and the second component is a receiver detecting the code when the first component and the second component are moved in proximity to one another due to movement of the carriage along the rail.

In yet another aspect, a kit of parts for retrofitting a rail-mounted lift system for automatically determining a location of an assembly along a length of a rail includes a locating system having a first component and a second component. One of the first component and the second component is a transmitter that provides a code corresponding to a predetermined location and another one of the first component and the second component is a receiver. The kit further includes network interface hardware communicatively coupled to the receiver, a server computing device communicatively coupled to the network interface hardware, and instructions for coupling the first component to a carriage or a lift unit of the rail-mounted lift system, coupling the second component on or near a rail of the rail-mounted lift system, and positioning the first component and the second component such that, when the first component and the second component are moved in proximity to one another due to movement of the carriage along the rail, a code is transmitted between the first component and the second component.

The invention will now be further described by way of example with reference to the accompanying drawings, in which:
FIG. 1 schematically depicts an illustrative rail-mounted lift system according to one or more embodiments described herein;
FIG. 2 schematically depicts an exploded view of various components of an interconnection between an illustrative carriage and an illustrative rail of a rail-mounted lift system according to one or more embodiments shown and described herein;
FIG. 3 schematically depicts a cross section of an illustrative rail of the rail-mounted lift system according to one or more embodiments described herein;
FIG. 4 schematically depicts a cross section of a rail-mounted lift system according to one or more embodiments shown and described herein;
FIG. 5 schematically depicts an illustrative hand control component of an assembly of a rail-mounted lift system according to one or more embodiments shown and described herein;
FIG. 6 depicts a block diagram of the electrical interconnectivity of components within an illustrative assembly of a rail-mounted lift system according to one or more embodiments shown and described herein;
FIG. 7A depicts a block diagram of illustrative internal components within an assembly of a rail-mounted lift system according to one or more embodiments shown and described herein;
FIG. 7B depicts a block diagram of illustrative internal components of a receiver of a locating system located on or near a rail according to one or more embodiments shown and described herein;
FIG. 8 schematically depicts an illustrative lift communications network according to one or more embodiments shown and described herein;
FIG. 9 schematically depicts a top-down view of an illustrative space in which a rail-mounted lift system may operate according to one or more embodiments shown and described herein;
FIG. 10 depicts a flow diagram of an illustrative overview method of transmitting data pertaining to an assembly of a rail-mounted lift system over a network according to one or more embodiments shown and described herein;
FIG. 11 depicts a flow diagram of an illustrative method of detecting the presence of an assembly of a rail-mounted lift system according to one or more embodiments shown and described herein;
FIG. 12 depicts a flow diagram of an illustrative method of determining an identity and a location of an assembly of a rail-mounted lift system within a space according to one or more embodiments shown and described herein;
FIG. 13 depicts a flow diagram of an illustrative method of pairing a user and a subject to an assembly according to one or more embodiments shown and described herein; and
FIG. 14 depicts a flow diagram of an illustrative method of establishing communications between an assembly and a central server according to one or more embodiments shown and described herein.

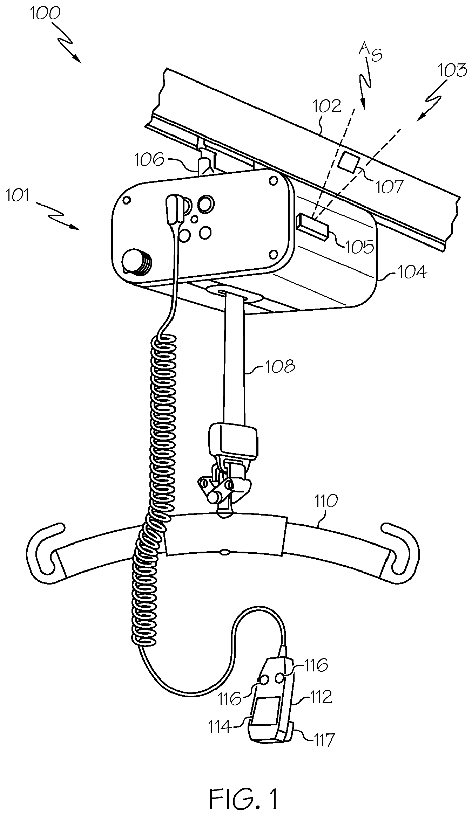

FIG. 1 generally depicts one embodiment of a rail-mounted lift system. The rail-mounted lift system generally includes a lift unit slidably coupled to a rail via a carriage. The rail extends along a ceiling of a space, such as various rooms and corridors of a hospital or other medical facility. Also included in FIG. 1 is a sensing system that generates and senses a coded signal that corresponds to a location of the carriage on the rail. The rail-mounted lift system also includes a hand control component that is used for controlling the lift system, associating a user and/or a subject with the rail-mounted lift system, and/or registering the rail-mounted lift system on a network. Using the components shown in FIG. 1, the rail-mounted lift system can be accurately tracked by a server computing device or the like because the movement of the lift along the rails and the specific positioning of the components of the sensing system ensures appropriate alignment when the rail-mounted lift system moves, and do not require human intervention to ensure appropriate alignment for functionality. The lift system and the various components of the lift system will be described in more detail herein with specific reference to the appended figures.

Referring now to FIG. 1, the rail-mounted lift system 100 generally includes an assembly 101 coupled to a rail 102. More specifically, the assembly 101 includes a lift unit 104 that is slidably coupled to a rail 102 via a carriage 106. The lift unit 104 may be used to support and/or lift a subject with a lifting strap 108 which is coupled to a motor contained within the lift unit 104. The motor facilitates extending or retracting the lifting strap 108 from the lift unit 104, thereby raising and lowering a subject attached to the lifting strap 108.

In the embodiment of the rail-mounted lift system 100 shown in FIG. 1, a subject may be attached to the lifting strap 108 with a sling bar 110 or a similar accessory attached to the lifting strap 108. More specifically, the sling bar 110 or a similar accessory may be attached to a harness or sling in which the subject is positioned, thereby facilitating the lifting operation.

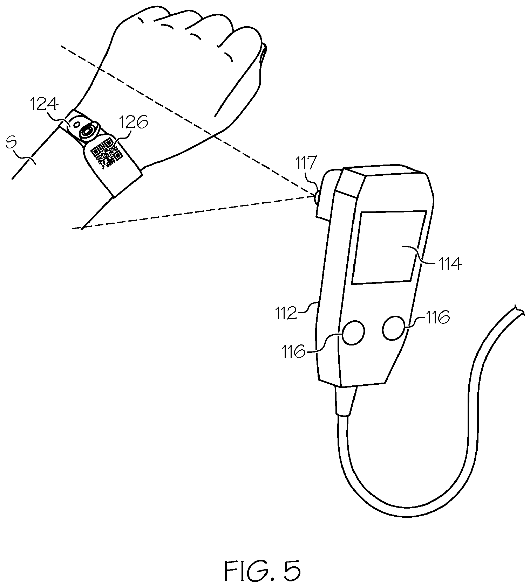

Various components of the assembly 101, such as the lift unit 104 and/or components thereof, may be operated with a hand control unit 112 that is communicatively coupled to the lift unit 104. In the embodiment shown in FIG. 1, the hand control unit 112 is directly wired to the lift unit 104. However, it should be understood that, in other embodiments, the hand control unit 112 may be wirelessly coupled to the lift unit 104 to facilitate remote operation of the assembly 101. In some embodiments, the hand control unit 112 may include a display 114, one or more user interface controls 116, and/or a sensing device 117. Additional details regarding the hand control unit 112 will be discussed herein with respect to FIG. 5.

Still referring to the embodiment of the rail-mounted lift system 100 shown in FIG. 1, a locating system 103 is coupled to one or more components of the rail-mounted lift system 100. The locating system 103 generally includes a first component 105 and a second component 107. The first component 105 and the second component 107 function as a receiver and a transmitter. That is, the first component 105 may be a receiver or a transmitter and the second component 107 may be a transmitter or a receiver. For example, in embodiments where the first component 105 is a receiver, the second component 107 may be a transmitter. Additionally, in embodiments where the first component 105 is a transmitter, the second component 107 may be a receiver.

The first component 105 and the second component 107, when configured as a transmitter, are generally any device or component that transmits, displays, or otherwise provides an encoded signal that is obtainable or otherwise receivable by the receiver. Accordingly, the first component 105 and the second component 107, when configured as a receiver, are generally any device or component that obtains or otherwise receives the encoded signal from the transmitter. The transmitter may be a passive device such as a printed code (such as a barcode, a QR code, or the like), or may be an active device such as an infrared (IR) beacon, a radio frequency identification (RFID) emitter or tag, a wireless transmitter, or the like. The transmitter is printed, attached, or otherwise affixed to an area on or around the rail 102 or a portion of the assembly 101, such as the lift unit 104 or the carriage 106. In embodiments where the transmitter is an RFID emitter or tag affixed to the rail 102, the RFID emitter or tag may be affixed in such a manner that the RFID signal is not propagated through the rail 102 (e.g., by coupling the RFID emitter or tag to an insulation device or the like). In addition, the receiver may be an imaging device, an IR receiver, an RFID detector (e.g., an electromagnetic field generator), a wireless receiver (e.g., a radio utilizing a wireless technology standard such as Bluetooth or 802.11x), or the like. The receiver may be attached to or integrated with a component of the assembly 101, such as, for example, the lift unit 104 or the carriage 106, or may be attached or integrated on or near the rail 102. The transmitter and the receiver may further be positioned or otherwise arranged such that the encoded signal provided by the transmitter is received by the receiver when the transmitter and the receiver pass one another due to movement of the carriage 106 along the rail 102, as described in greater detail herein. For example, the transmitter and the receiver may be arranged such that a line-of-sight alignment is achieved between the transmitter and the receiver when the carriage 106 passes a particular location along the rail 102.

In the embodiment depicted in FIG. 1, the first component 105 is coupled to the lift unit 104 and the second component 107 is positioned on the rail 102. However, these locations are merely illustrative, and the first component 105 and the second component 107 may be located elsewhere with respect to the rail-mounted lift system 100 (or components thereof) in other embodiments. Solely for the purposes of explanation herein, the embodiment of FIG. 1 depicts the first component 105 coupled to the lift unit 104 and configured as a receiver, and the second component 107 positioned on the rail 102 and configured as a transmitter.

The portion of the locating system 103 that functions as the receiver (e.g., the first component 105 or the second component 107) may sense a sensed area Aₛ that generally encompasses an area adjacent to the receiver. That is, the sensed area Aₛ may be an area completely surrounding the receiver in some embodiments. In other embodiments, the sensed area Aₛ may be an area encompassed by the field of view of the receiver, such as when the receiver is an imaging device or an IR receiver. In the embodiment depicted in FIG. 1, the sensed area Aₛ may generally be an area bound by the dashed lines, which extends from the lift unit 104 to a portion of the rail 102.

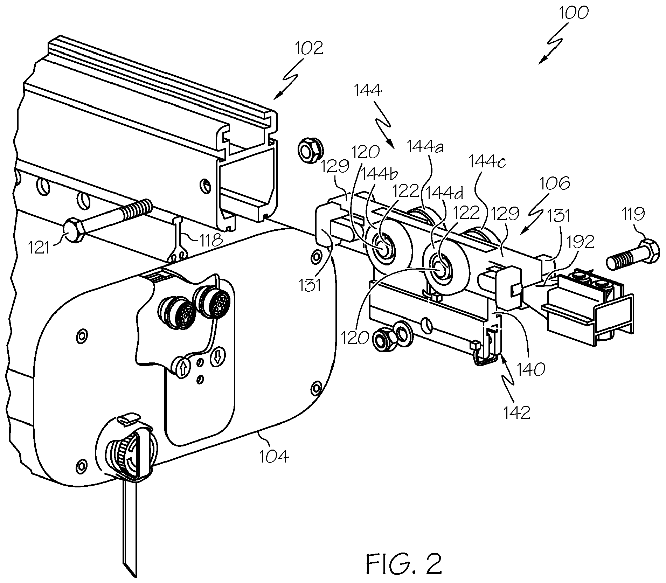

Referring now to the exploded view of the rail-mounted lift system 100 schematically depicted in FIG. 2, the lift unit 104 is mechanically coupled to a carriage 106 which facilitates slidably positioning the lift unit 104 along the rail 102. In the embodiments of the lift unit 104 described herein, the lift unit 104 includes a connection rail 118 which is mounted to a top surface of the lift unit 104. The connection rail 118 facilitates connecting and securing the lift unit 104 to the carriage 106. In the embodiment of the lift unit 104 shown in FIG. 2, the connection rail 118 has a T-shaped configuration and the carriage 106 has a receiving slot 142 with a complimentary configuration for receiving the connection rail 118. The carriage 106 may be secured to the connection rail 118 with a fastener 119, such as a bolt and nut as depicted in FIG. 2, which extends transversely through openings in the carriage 106 and a corresponding opening in the connection rail 118.

In embodiments, the carriage 106 generally includes a carriage body 140 to which a plurality of support wheels 144a, 144b, 144c, and 144d (collectively, support wheels 144) are rotatably attached for supporting the carriage 106 in the rail 102. The support wheels 144 facilitate positioning the carriage 106 and lift unit 104 along the length of the rail 102. In the embodiments described herein, the carriage 106 is depicted with four support wheels 144. However, it is contemplated that the carriage 106 may be constructed with fewer than four (4) support wheels 144. For example, in some embodiments, the carriage 106 may be constructed with one or two support wheels 144 (e.g., a pair of support wheels 144). Accordingly, it should be understood that the carriage 106 includes at least one support wheel 144. The support wheels 144 are positioned on axles 120 which extend transversely through the carriage body 140. Each support wheel 144 is secured to the axle 120 with a fastener, such as retaining clips 122, such that the support wheels 144 are rotatable on the axle 120.

In the embodiment of the carriage 106 depicted in FIG. 2, the support wheels 144 are passive (i.e., the support wheels 144 are not actively driven with a motor or a similar drive mechanism) and the lift unit 104 is manually traversed along the rail 102 (e.g., such as when a user pushes or pulls the lift unit 104 along the rail 102). However, in alternative embodiments (not shown), the support wheels 144 may be actively driven such as when the support wheels 144 are coupled to a motor or a similar mechanism. In such embodiments, the drive mechanism may be communicatively coupled to a hand control (such as hand control unit 112 shown in FIG. 1) which actuates the drive mechanism and facilitates traversing the lift unit 104 along the rail 102 with the drive mechanism.

The carriage 106 may further include bumper assemblies 129 positioned on either end of the carriage body 140 in some embodiments. The bumper assemblies 129 may be attached to the carriage body 140 and secured in place with the axles 120 which extend transversely through the carriage body 140. The bumper assemblies 129 may include rubber end caps 131 which cushion the carriage 106 when the carriage 106 encounters an end stop 192 located at either end of the rail 102, the end stops 192 being secured in the rail 102 by a fastener 121, which may be a bolt and a nut in some embodiments. In the embodiments of the carriage 106 described herein, the bumper assemblies 129 are optional and, as such, it should be understood that the carriage 106 may be constructed without the bumper assemblies 129.

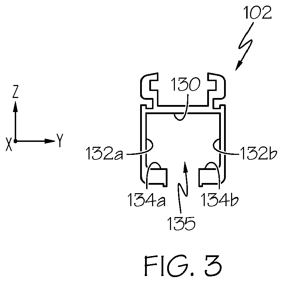

Referring to FIGS. 2 and 3, the rail-mounted lift system 100 further includes a rail 102 in which the carriage 106 is slidably disposed for relative movement to the rail 102. Accordingly, it should be understood that, when the lift unit 104 is mechanically coupled to the carriage 106, the lift unit 104 may be traversed along the rail 102 with the carriage 106. In the embodiment of the rail 102 shown in FIG. 3, the rail 102 is generally formed from a metallic material, such as aluminum, an aluminum alloy, or a similar metallic material, particularly materials that do not interfere with the functionality of the second component 107 (FIG. 1). Still referring to FIGS. 2 and 3, the rail 102 generally includes an upper portion 130, a first sidewall 132a integrally formed with the upper portion 130, and a second sidewall 132b integrally formed with the upper portion 130. The upper portion 130, first sidewall 132a and second sidewall 132b are oriented such that the upper portion 130, first sidewall 132a and second sidewall 132b form a carriage support channel 135 in which the carriage 106 is slidably disposed. To that end, the first sidewall 132a further includes a first support flange 134a which extends from the first sidewall 132a into the carriage support channel 135 and the second sidewall 132b further includes a second support flange 134b which extends from the second sidewall 132b into the carriage support channel 135. In the embodiments described herein, the first support flange 134a and the second support flange 134b are generally opposed to one another and lie in a common horizontal plane (e.g., the x-y plane in the coordinate axes shown in FIG. 3). The first support flange 134a and the second support flange 134b may also be substantially parallel with the upper portion 130 of the rail 102. However, it should be understood that other configurations of the support flanges 134a, 134b and the upper portion 130 of the rail 102 are also contemplated. For example, in an alternative embodiment, the support flanges 134a, 134b may be upwardly angled with respect to the horizontal plane. Moreover, it should be understood that the structure of the rail 102 depicted in FIGS. 2 and 3 is illustrative and that other rail configurations are contemplated.

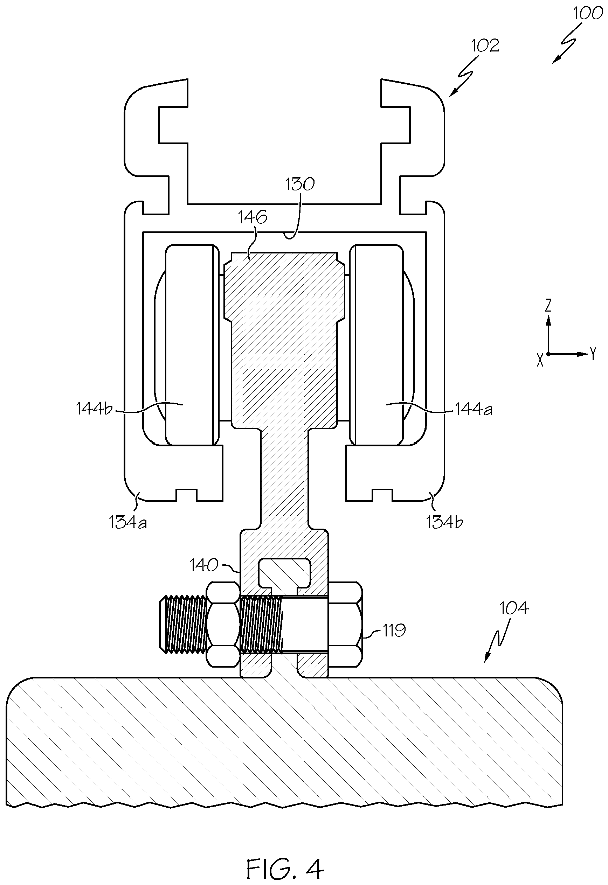

The construction of the rail-mounted lift system 100 will now be described with specific reference to the exploded view of the rail-mounted lift system 100 shown in FIG. 2 and the cross section of the assembled rail-mounted lift system 100 shown in FIG. 4. As shown in FIGS. 2 and 4, the carriage 106 is installed on the lift unit 104 by inserting the connection rail 118 into the corresponding receiving slot 142 of the carriage 106 and securing the carriage 106 on the connection rail 118 with a fastener 119. The lift unit 104 with attached carriage 106 is then suspended from the rail 102 by positioning the carriage 106 in the rail 102 such that the support wheels 144a, 144b are slidably engaged with the first support flange 134a and the second support flange 134b, respectively.

Referring to FIGS. 1 and 5, the hand control unit 112 includes one or more components that provide functionality for using the lift unit 104 (e.g., causing the motor within the lift unit 104 to extend or retract the lifting strap 108), authenticating a user, pairing the lift unit 104 with a particular subject S, and/or registering the assembly 101 with a network. The hand control unit 112 may include, for example, a display 114, one or more user interface controls 116, and/or a sensing device 117. The display 114 is generally any liquid crystal display (LCD), light emitting diode (LED) display, electronic ink (e-ink) display, or the like that can display information to a user. In some embodiments, the display 114 may be configured as an interactive display that can receive user inputs (e.g., a touch screen display or the like). The one or more user interface controls 116 may be hardware components that receive inputs from a user and transmit signals corresponding to the inputs, such as a keyboard, a mouse, a joystick, a touch screen, a remote control, a pointing device, a video input device, an audio input device, a haptic feedback device, and/or the like. In some embodiments, the display 114 and one or more of the user interface controls 116 may be combined into a single device, such as a touchscreen display or the like. The display 114 and/or the one or more user interface controls 116 may be used, for example, to allow a user to manually input information pertaining to an identity of the user, an identity of a subject, a location or sub-location of the assembly 101, to provide instructions for programming and/or pairing the assembly 101 with one or more other components, and/or the like.

The sensing device 117 of the hand control unit 112 contains hardware for sensing a code and transmitting data corresponding to the code. Illustrative hardware includes, but is not limited to, an imaging device, an IR receiver, an RFID detector (e.g., an electromagnetic field generator), a wireless receiver (e.g., a radio utilizing a wireless technology standard such as Bluetooth or 802.11x), or the like. In the embodiment depicted in FIG. 5, the sensing device 117 is an imaging device that is adapted to read a barcode 126 (or any other symbology, QR code, or the like) that is printed on an identification bracelet 124 of a subject S. The sensing device 117 may also be used for sensing other codes, such as a personnel ID badge code, a code used for configuring the assembly 101 for use on a network and/or the like.

The barcode 126 located on the identification bracelet 124 is encoded with information that, when transmitted to a remote server, can be used to identify the subject S, determine the location of subject S, confirm that the proper equipment is being used for the subject S, and/or the like. For example, the information can be used to determine whether the lift unit 104 is rated to support the weight of the subject S, whether the correct accessories such as the sling bar 110 are attached to the lift unit 104, and/or the like. While the barcode 126 is depicted in FIG. 5, it should be understood that the code corresponding to the subject S may be provided by any other means. For example, an RFID tag, an infrared tag, and/or any other technology, particularly technologies used in hospital and medical settings, may be used to convey coded information corresponding to the identity of the subject S.

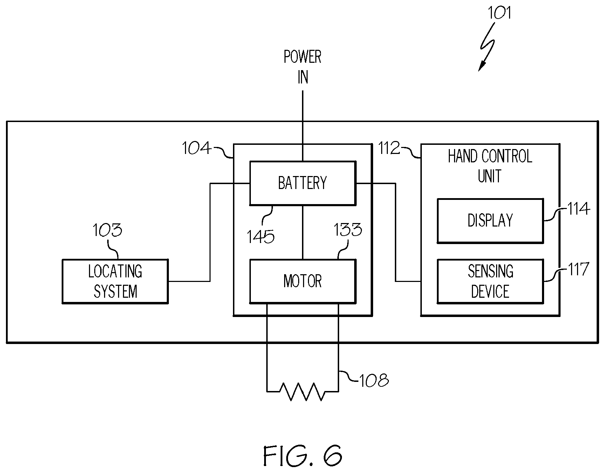

Various components of the assembly 101 may be powered by a power source. Referring to FIGS. 1 and 6, the power source may be a battery 145 in some embodiments. As such, various power components of the assembly 101, such as components contained within the lift unit 104, may be electrically coupled to the battery 145. For example, the battery 145 may be electrically coupled to the hand control unit 112 and/or various components thereof (e.g., the display 114 and/or the sensing device 117), the locating system 103 and/or a component thereof (e.g., the first component 105), and/or the like. In the embodiment depicted in FIG. 6, the assembly 101 includes the battery 145 which is housed in the lift unit 104 and electrically coupled to a motor 133 that facilitates movement of the lifting strap 108, thereby providing power to the motor 133. However, it should be understood that, in other embodiments, the lift unit 104 may be constructed without the battery, such as when the motor 133 is directly coupled to a power source that is external to the assembly 101, such as conductors or the like located on or adjacent to the rail 102. For example, the carriage 106 may include conductive rollers that are electrically coupled to electrical conductor track on or within the rail 102 that supplies electrical power to the carriage 106 and the various other components of the assembly 101. In another example, the components of the assembly 101 may be electrically coupled to an inertial power generator located on the assembly 101. The inertial power generator may supply electrical power to the battery 145 and/or the other components of the assembly 101 when the assembly 101 is moved along the rail 102. That is, movement of the assembly 101 causes electrical power to be generated via the inertial power generator. Other means of providing electrical power to the rail-mounted lift system 100 and the components thereof are also contemplated and included within the scope of the present disclosure.

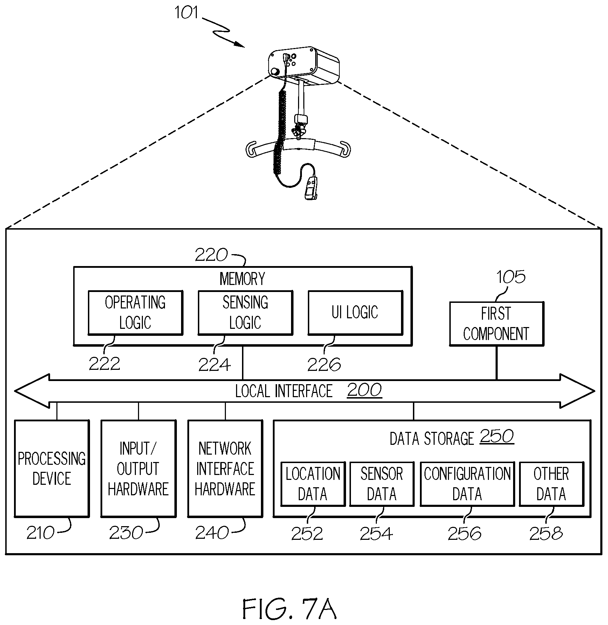

In addition to the battery 145, various other internal components may also be contained within the assembly 101 in some embodiments. FIG. 7A depicts such illustrative internal components that are communicatively coupled to one another to provide the functionality of the rail-mounted lift system 100 described herein. As shown in FIG. 7A, the assembly 101 may further include a local interface 200 (e.g., a bus) that communicatively interconnects the various components, including, but not limited to, a processing device 210, memory 220, input/output hardware 230, network interface hardware 240, a data storage device 250, and/or the first component 105 of the locating system 103.

The processing device 210, such as a computer processing unit (CPU), may be the central processing unit of the assembly 101, performing calculations and logic operations required to execute a program. The processing device 210, alone or in conjunction with one or more of the other elements disclosed in FIG. 7A, is an illustrative processing device, computing device, processor, or combination thereof, as such terms are used in this disclosure.

Referring to FIGS. 1 and 7A, the memory 220, such as read only memory (ROM) and random access memory (RAM), may constitute illustrative memory devices (i.e., non-transitory, processor-readable storage media). Such memory 220 may include one or more programming instructions thereon that, when executed by the processing device 210, cause the processing device 210 to complete various processes, such as the processes described herein. Optionally, the program instructions may be stored on a tangible computer-readable medium such as a digital disk, flash memory, a memory card, a USB drive, an optical disc storage medium (e.g., Blu-ray™, CD, DVD), and/or other non-transitory processor-readable storage media.

In some embodiments, the program instructions contained on the memory 220 may be embodied as a plurality of software modules, where each module provides programming instructions for completing one or more tasks. For example, as shown in FIG. 7A, the memory 220 may contain one or more of operating logic 222, sensing logic 224, and user interface (Ul) logic 226. The operating logic 222 may include an operating system and/or other software for managing components of the assembly 101. The sensing logic 224 may generally include programming instructions for directing operation of the locating system 103 or a portion thereof (such as the first component 105). For example, the sensing logic 224 may direct the first component 105 to turn on/off, to collect data corresponding to the code provided by the second component 107, and/or the like. The Ul logic 226 may generally include programming instructions for interfacing with a user via the hand control unit 112. It should be understood that the various logic modules described herein with respect to FIG. 7A are merely illustrative, and that other logic modules, including logic modules that combine the functionality of two or more of the modules described hereinabove, may be used without departing from the scope of the present application.

Still referring to FIGS. 1 and 7A, the data storage device 250, which may generally be a storage medium that is separate from the memory 220, may contain a data repository for storing electronic data and/or the like relating to the location of the assembly 101, an identification of the assembly 101, a sensed code, configuration settings, and/or the like. The data storage device 250 may be any physical storage medium, including, but not limited to, a hard disk drive (HDD), memory, removable storage, and/or the like. While the data storage device 250 is depicted as a local device, it should be understood that the data storage device 250 may be a remote storage device that is remotely located from the assembly 101, such as, for example, a server computing device or the like.

Illustrative data that may be contained within the data storage device 250 may include, for example, location data 252, sensor data 254, configuration data 256, and/or other data 258. Location data 252 may include, for example, information pertaining to a location and/or an identity of the assembly 101, such as a code corresponding to the last stored location of the assembly 101 or the like. The sensor data 254 may include, for example, data relating to information that is sensed by the receiver of the locating system 103 (e.g., the first component 105 or the second component 107), including a code that is usable by a server computing device to identify the location of the assembly 101, a code that is usable by a server computing device to identify an associated subject, a code that is usable by a server computing device to identify an associated user, and/or the like. The configuration data 256 may include, for example, data relating to configuring the assembly 101 such that the assembly 101 can be communicatively coupled to a remote server, as described in greater detail herein.

The input/output hardware 230 may generally include hardware contained within the hand control unit 112. That is, the display 114 and/or the one or more user interface controls 116 may be communicatively coupled to the input/output hardware 230 such that one or more user inputs received via the display 114 and/or the one or more user interface controls 116 are transmitted via the input/output hardware 230 and one or more outputs to be displayed to the user are transmitted to the display 114 via the input/output hardware 230. In some embodiments, the sensing device 117 may also be communicatively coupled to the input/output hardware 230 such that data corresponding to a sensed code (e.g., image data containing a sensed code) are transmitted via the input/output hardware 230.

The network interface hardware 240 may generally provide the assembly 101 with an ability to interface with one or more external components, such as, for example, an external computing device, a remote server, and/or the like that is external to the assembly 101. Communication with external devices may occur using various communication ports (not shown). An illustrative communication port may be attached to a communications network, such as the Internet, an intranet, a local network, a direct connection, and/or the like.

It should be understood that in some embodiments, the input/output hardware 230 and the network interface hardware 240 may be combined into a single device that allows for communications with other devices, regardless of whether such other devices are located within the assembly 101.

It should be understood that the components illustrated in FIG. 7A are merely illustrative and are not intended to limit the scope of this disclosure. More specifically, while the components in FIG. 7A are illustrated as residing within the assembly 101, this is a nonlimiting example. In some embodiments, one or more of the components may reside external to the assembly 101. Similarly, one or more of the components may be embodied in other devices not specifically described herein.

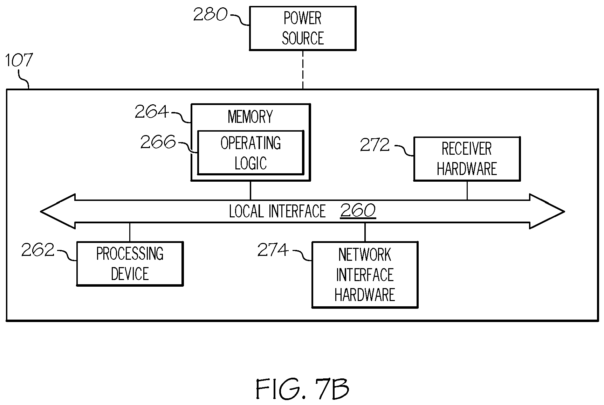

In embodiments where the second component 107 that is coupled on or near the rail 102 is a receiver, the second component 107 may have internal components that generally function to transmit data corresponding to a received code. FIG. 7B depicts illustrative internal components of the second component 107 in such embodiments. As shown in FIG. 7B, the second component 107 may include a local interface 260 (e.g., a bus) that communicatively interconnects the various components, including, but not limited to, a processing device 262, memory 264, receiver hardware 272, and/or network interface hardware 274. In some embodiments, one or more of the components of the second component 107 depicted in FIG. 7B may be electrically coupled to a power source 280, such as a battery or an AC power source.

The processing device 262, such as a computer processing unit (CPU), may be the central processing unit of the second component 107, performing calculations and logic operations required to execute a program. The processing device 262, alone or in conjunction with one or more of the other elements disclosed in FIG. 7B, is an illustrative processing device, computing device, processor, or combination thereof, as such terms are used in this disclosure.

The memory 264, such as read only memory (ROM) and random access memory (RAM), may constitute illustrative memory devices (i.e., non-transitory, processor-readable storage media). Such memory 264 may include one or more programming instructions thereon that, when executed by the processing device 262, cause the processing device 262 to complete various processes, such as the processes described herein. Optionally, the program instructions may be stored on a tangible computer-readable medium such as a digital disk, flash memory, a memory card, a USB drive, an optical disc storage medium (e.g., Blu-ray™, CD, DVD), and/or other non-transitory processor-readable storage media. In some embodiments, the program instructions contained on the memory 264 may be embodied as a plurality of software modules, where each module provides programming instructions for completing one or more tasks. For example, as shown in FIG. 7B, the memory 264 may contain operating logic 222. The operating logic 222 may include an operating system and/or other software for managing components of the second component 107, including software for directing the receiver hardware 272 to receive a code, generating data corresponding to the code, and/or directing the network interface hardware 274 to transmit the data. It should be understood that the logic module described herein with respect to FIG. 7B are merely illustrative, and that other logic modules, including logic modules that combine the functionality of two or more of the modules described hereinabove, may be used without departing from the scope of the present application.

The receiver hardware 272 generally encompasses any hardware components that can be used to receive a code from the first component 105 of the locating system 103 (FIG. 1). Still referring to FIG. 7B, the receiver hardware 272 may be an imaging device, an IR receiver, an RFID detector (e.g., an electromagnetic field generator), a wireless receiver (e.g., a radio utilizing a wireless technology standard such as Bluetooth or 802.11x), or the like, as described herein.

The network interface hardware 274 may generally provide the second component 107 with an ability to interface with one or more external components, such as, for example, an external computing device, a remote server, and/or the like that is external to the second component 107. Communication with external devices may occur using various communication ports (not shown). An illustrative communication port may be attached to a communications network, such as the Internet, an intranet, a local network, a direct connection, and/or the like.

It should be understood that the components illustrated in FIG. 7B are merely illustrative and are not intended to limit the scope of this disclosure. More specifically, while the components in FIG. 7B are illustrated as residing within the second component 107, this is a nonlimiting example. In some embodiments, one or more of the components may reside external to the second component 107. Similarly, one or more of the components may be embodied in other devices not specifically described herein.

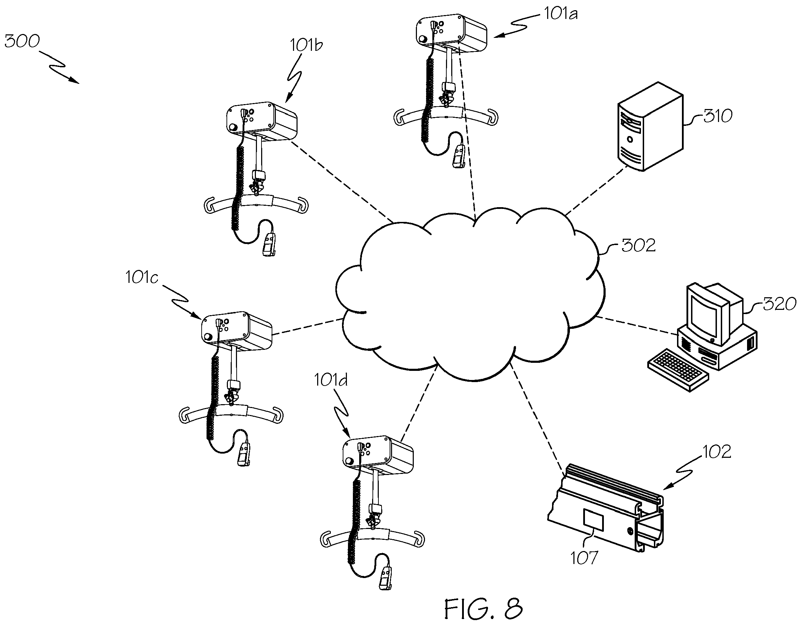

Referring again to FIG. 1, the assembly 101 may generally be communicatively coupled to one or more components external to the assembly 101 such that the sensed codes and other information received by the assembly 101 (or components thereof) can be transmitted for the purposes of determining a location of the assembly 101, authenticating a user to use the assembly 101, and/or confirming that a particular subject can be lifted with the assembly 101. Similarly, in some embodiments, the second component 107 may be communicatively coupled to one or more components external to the second component 107 such that the sensed codes and received by the second component 107 (or components thereof) can be transmitted for the purposes of determining a location of the assembly 101. That is, the assembly 101 and/or the second component 107 may be coupled to one or more components in a system via a communications network. FIG. 8 depicts a system 300 comprising an illustrative lift communications network 302 that communicatively couples one or more components.

As illustrated in FIG. 8, the lift communications network 302 may include a wide area network (WAN), such as the Internet, a local area network (LAN), a mobile communications network, a public service telephone network (PSTN), a personal area network

(PAN), a metropolitan area network (MAN), a virtual private network (VPN), and/or another network. The lift communications network 302 may generally be configured to electronically connect one or more devices such as computing devices and/or components thereof. Illustrative devices may include, but are not limited to, one or more assemblies 101a, 101b, 101c, 101d, a server computing device 310, a user computing device 320, and/or the second component 107 of the locating system 103 (FIG. 1).

Still referring to FIG. 8, the user computing device 320 may generally be used as an interface between a user and the other components connected to the lift communications network 302. Thus, the user computing device 320 may be used to perform one or more user-facing functions, such as receiving one or more inputs from a user or providing information to the user. Accordingly, the user computing device 320 may include at least a display and/or input hardware, as described in greater detail herein. In the event that the server computing device 310 requires oversight, updating, and/or correction, the user computing device 320 may be configured to provide the desired oversight, updating, and/or correction. The user computing device 320 may also be used to input additional data into a corpus of data stored on the server computing device 310. For example, the user computing device 320 may contain software programming or the like that allows a user to view a list of subjects, view a list of the one or more assemblies 101a-101d and their respective locations, provide information regarding subjects, provide information regarding users authorized to operate the one or more assemblies 101a-101d, change settings, and/or the like.

The server computing device 310 may receive data from one or more sources (e.g., components of each of the one or more assemblies 101a-101d), analyze received data (e.g., determine a location of an assembly 101, an associated subject, and authorized user, and/or the like), generate data, store data, index data, search data, and/or provide data to the user computing device 320 and/or each of the one or more assemblies 101a-101d (or components thereof). More specifically, the server computing device 310 may receive a code from an assembly 101, determine a location that corresponds to the code, record the location, receive information corresponding to a subject from an assembly 101, determine an identity of the subject and verify the assembly 101 can be used with the subject, receive information corresponding to a user, determine an identity of the user and verify that the user is authorized to use the assembly 101, and/or the like, as described in greater detail herein. In some embodiments, the server computing device 310 may employ one or more software programs that are used to connect to one or more external devices. One illustrative software program includes, but is not limited to, the NaviCare® software application provided by Hill-Rom Services Inc. (Chicago, IL).

It should be understood that while the user computing device 320 is depicted as a personal computer and the server computing device 310 is depicted as a server, these are nonlimiting examples. In some embodiments, any type of computing device (e.g., mobile computing device, personal computer, server, cloud-based network of devices, etc.) may be used for any of these components. Additionally, while each of these computing devices is illustrated in FIG. 8 as a single piece of hardware, this is also merely an example. Each of the user computing device 320 and the server computing device 310 may represent a plurality of computers, servers, databases, components, and/or the like.

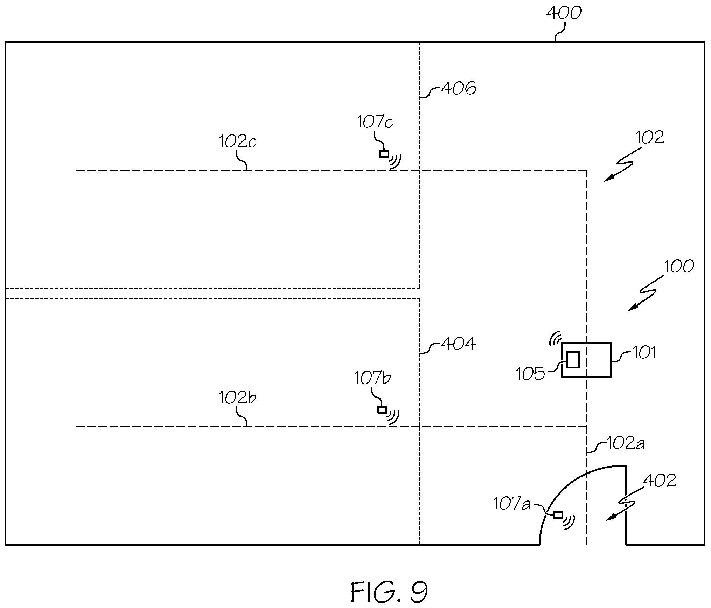

FIG. 9 depicts an illustrative space in which the rail-mounted lift system 100 may operate. Referring to FIGS. 1 and 9, in operation, the assembly 101 can be moved along the rail 102 into a space 400, such as a room. As such, the rail 102, which is mounted on the ceiling of the space 400, may extend out of the space 400 through an opening 402, such as a doorway or the like. In the embodiment depicted in FIG. 9, the space 400 may be divided into a plurality of subspaces 404, 406 and the rail 102 may extend into each of these subspaces 404, 406. Illustrative examples of a space 400 having a plurality of subspaces 404, 406 may include, but are not limited to, shared rooms, a pre-operating room, a therapy room, or the like. In order to extend into each subspace 404, 406, the rail 102 may have a plurality of rail segments 102a, 102b, 102c that are interconnected with one another, where each one of the plurality of rail segments 102a, 102b, 102c extends into a respective portion of the space 400, such as a subspace 404, 406 thereof. In addition, the plurality of rail segments 102a, 102b, 102c are interconnected such that when the assembly 101 is slidably moved along the rail 102, a user guiding the assembly 101 along the rail 102 can move the assembly 101 between each of the rail segments 102a, 102b, 102c as needed to enter an area of the space 400, such as a subspace 404, 406 thereof.

Referring to FIGS. 1, 8, and 9, as the assembly 101 traverses the rail 102 within the space 400, the assembly 101 passes the second component 107 of the locating system 103, which is used to transmit a code to the server computing device 310 corresponding to the location of the assembly 101. In some embodiments, a plurality of second components 107a, 107b, 107c may be placed in an area of the space 400, such as near the opening 402 or in the subspaces 404, 406 of the space 400. As such, the location of the assembly 101 can be narrowed down to a particular area of the space 400 (e.g., one of the subspaces 404, 406).

As previously described herein, the second components 107a-107c of the locating system 103 may each be a transmitter or a receiver. In embodiments where the second components 107a-107c are transmitters, the second components 107a-107c may transmit a unique code corresponding to their respective locations, which, when detected by the first component 105 on the assembly 101, causes the assembly 101 to transmit data to the server computing device 310 that corresponds to the detected code so that the server computing device 310 can determine the location of the assembly 101 based on the sensed unique code. For example, a transmitter located near the opening 402 to the space 400 (e.g., second component 107a) may transmit a code, which, when detected by the first component 105 when the assembly 101 moves along the rail 102 through the opening 402 into the space 400, causes the assembly 101 (and more specifically, the network interface hardware 240 thereof (FIG. 7A)) to transmit data to the server computing device 310 that corresponds to the code transmitted by second component 107a. Still referring to FIGS. 1, 8, and 9, in another example, a transmitter located near rail segment 102b that enters into subspace 404 of the space 400 (e.g., second component 107b) may transmit a code, which, when detected by the first component 105 when the assembly 101 moves along rail segment 102b into subspace 404, causes the assembly 101 (and more specifically, the network interface hardware 240 thereof (FIG. 7A)) to transmit data to the server computing device 310 that corresponds to the code transmitted by second component 107b.

In embodiments where the second components 107a-107c are receivers, the second components 107a-107c may receive a code corresponding to an identity of the assembly 101 that is transmitted by the first component 105 coupled to the assembly 101 when the assembly 101 traverses the rail 102 past the respective second components 107a-107c. Upon receiving the code, the second components 107a-107c may transmit data to the server computing device 310 that corresponds to the code received from the first component 105, which allows the server computing device 310 to determine the location of the assembly 101 because the location of the respective second components 107a-107c are stored and the identity of the assembly 101 is included in the code transmitted by the first component 105. For example, a receiver located near rail segment 102c that enters into subspace 406 of the space 400 (e.g., second component 107c) may receive a code from the first component 105 coupled to the assembly 101 when the assembly 101 moves along rail segment 102c into subspace 406. In turn, second component 107c transmits data to the server computing device 310 that corresponds to the code transmitted by the first component 105.

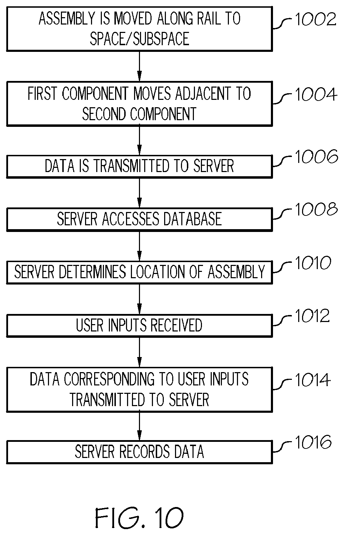

FIG. 10 depicts a flow diagram of the processes described above with respect to FIG. 9. Referring to FIGS. 1, 3, 7A-7B, and 8-10, the assembly 101 is moved along the rail 102 at block 1002. More specifically, the carriage 106 of the assembly 101 that is slidably disposed in the carriage support channel 135 of the rail 102 is moved until the assembly 101 is located in a desired space 400 (e.g., a room) or a subspace 404, 406 thereof. Movement is generally completed manually by a user, such as medical personnel or the like, by manually pushing or pulling on a portion of the assembly 101. In some embodiments, movement may be facilitated mechanically, such as by a motor or the like, to move along the rail 102 to the desired space 400.

At block 1004, the first component 105 moves adjacent to the second component 107 as the assembly 101 is moved along the rail. That is, the receiver portion of the locating system 103 (e.g., either the first component 105 or the second component 107), detects a code within a field of view thereof, an electromagnetic field generated thereby, an IR signal received, a wireless signal received, and/or the like. For example, if the receiver is an imaging device and the transmitter is a barcode, movement of the assembly 101 along the rail may cause the barcode to come into the field of view of the imaging device due to respective placement of the first component 105 and the second component 107 such that a code that is encoded by the barcode is received by the imaging device.

At block 1006, data corresponding to the received code is transmitted to the server computing device 310. That is, the receiver portion of the locating system 103 (e.g., the first component 105 or the second component 107, depending on configuration) generates data corresponding to the code (e.g., a data transmission including the code therein) and transmits the data to the server computing device 310. In some embodiments, the data may further include a time stamp or the like that indicates when the code was received, an identity of the receiver, and/or the like. Once received by the server computing device 310, the server computing device 310 accesses a database at block 1008. The database generally contains a look-up table or the like that corresponds individual codes to particular locations. For example, the database may associate code 1234 with location WXYZ. Thus, when the server computing device 310 searches the database for code 1234, location WXYZ will be discovered as being the location at which the assembly 101 is located. Accordingly the location of the assembly 101 is determined by the server computing device 310 at block 1010. Determining the location of the assembly 101 may include generating data that corresponds to the location of the assembly 101, the data identifying the particular location and/or the time at which the assembly 101 was located at the particular location.

In some embodiments, additional data provided through the hand control unit 112 may also be transmitted to the server computing device 310. That is, the user inputs are received at block 1012 (e.g., via the hand control unit 112 or a portion thereof) and the data corresponding to the user inputs are transmitted to the server computing device 310 at block 1014. As described in further detail herein, the additional data can be used to further determine the location of the assembly 101 (e.g., determine a sub space), associate the assembly 101 with a subject, associate the assembly 101 with a user, verify that the user is authorized to use the assembly 101, and/or verify that the equipment included with the assembly 101 is appropriate for use with the subject.

At block 1016, the server computing device 310 records the received data. That is, data corresponding to the location of the assembly 101, including data generated as a result of the processes described above with respect to blocks 1008 and 1010, may be stored for future retrieval. In addition, data corresponding to the inputs received via the hand control unit 112 may also be recorded. Such data may be used for the purposes of locating the assembly 101 at a particular time, both within a space and/or a subspace thereof, associating the assembly 101 with a subject and/or a user, verifying that the equipment included with the assembly 101 is appropriate for use with the subject, and/or verifying that the user is authorized to operate the assembly 101.

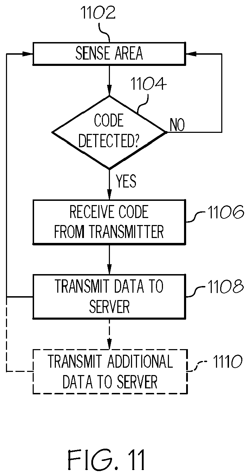

The particular processes with respect to obtaining and transmitting the code corresponding to a particular location are depicted in the flow diagram of FIG. 11. Referring now to FIGS. 1 and 11, the sensed area Aₛ is sensed at block 1102. That is, the portion of locating system 103 that is the receiver (e.g., the first component 105 or the second component 107) may be used to sense the sensed area Aₛ in a field of view thereof, generate an electromagnetic field or the like to sense the sensed area Aₛ, activate such that signals can be received within the sensed area Aₛ, and/or the like. In some embodiments, the receiver may be powered on to sense the sensed area Aₛ according to block 1102. Powering on the receiver may be completed, for example, by actuating a power switch or otherwise causing electrical power to be supplied to the receiver. In some embodiments, movement of the carriage 106 may generate electrical power that activates the receiver (e.g., when the receiver is the first component 105 of the locating system 103) using an inertial power generator integrated with the carriage 106.

At block 1104, a determination is made as to whether a code is detected. That is, if the receiver portion of the locating system 103 (e.g., the first component 105 or the second component 107) receives or otherwise detects an emitted signal, the determination may be made that a code has been detected. If no code has been detected, the process may return to block 1102 until a code is detected. That is, the receiver portion of the locating system 103 may continuously sense the sensed area Aₛ until a code is detected.

If the code is detected, the code is received from the transmitter portion of the locating system 103 (e.g., the first component 105 or the second component 107) at block 1106. That is, the encoded signal transmitted by the transmitter portion of the locating system 103 (e.g., the first component 105 or the second component 107) may be received to extract the code therefrom. In embodiments where the second component 107 is the receiver, the encoded signal may be received via the receiver hardware 272 of the second component 107 (FIG. 7B). Still referring to FIGS. 1 and 11, in some embodiments, receiving the code according to block 1106 may occur automatically as soon as it is detected.

Referring now to FIGS. 1, 7A-7B, 8, and 11, data containing the code is transmitted to the server computing device 310 at block 1108. That is, in embodiments where the receiver is the first component 105 of the locating system 103, the network interface hardware 240 of the assembly 101 may transmit the data via the lift communications network 302 to the server computing device 310. In embodiments where the receiver is the second component 107 of the locating system 103, the network interface hardware 274 of the second component 107 may transmit the data via the lift communications network 302 to the server computing device 310. In addition to the code, the data may also include additional information that is usable to track the assembly 101, such as, for example, a time stamp that indicates when the code was received (which generally corresponds to the time at which the assembly 101 passed the second component 107 of the locating system 103), information pertaining to the identity of the receiver portion of the locating system (e.g., the first component 105 or the second component 107), information pertaining to an identity of the assembly 101, and/or the like.

It should be understood that the processes described with respect to FIG. 11, particularly with respect to blocks 1102-1108, may be repeated continuously as the assembly 101 is moved along a length of the rail 102 such that the code is transmitted each time the assembly 101 passes one or more second components 107 at or near the rail 102. For example, if a user moves the assembly 101 from a first point to a second point, the rail 102 that extends between the first point and the second point may include or pass near one or more second components 107. Thus, the assembly, as it traverses the rail 102 from the first point to the second point, may pass each of the one or more second components 107. Accordingly the process described with respect to FIG. 11 may occur continuously such that a code corresponding to each of the one or more second components 107 is transmitted to the server computing device 310 each time the assembly 101 passes one of the one or more second components 107. Accordingly, the server computing device 310 can accurately track the movement of the assembly 101 and ensure that an up-to-date location of the assembly at any point in time is stored for retrieval.

In some embodiments, additional data may also be transmitted to the server computing device 310 at block 1110. That is, data that is received via the hand control unit 112, such as received encoded signals corresponding to an identity of a user and/or a subject, user inputs, and/or the like, may be transmitted to the server computing device 310. For example, data received via the sensing device 117 and/or the user interface controls may be transmitted by the network interface hardware 240 via the lift communications network 302 to the server computing device 310. It should be understood that the process described with respect to block 1110 is optional, and that the processes may only occur if an input is received via the hand control unit 112. In some embodiments, the process may optionally return to block 1102.

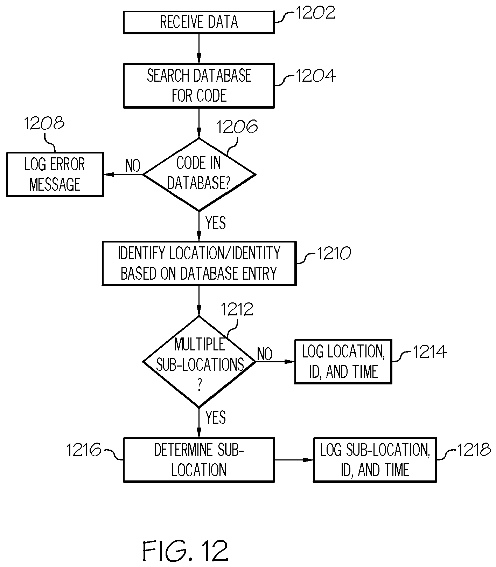

Once the server computing device 310 has received a transmission from the assembly 101 as described in FIG. 11, the server computing device 310 may complete one or more processes to determine the identity and location of the assembly 101 and record the identity and location of the assembly 101. FIG. 12 depicts a flow diagram of an illustrative method of determining the identity and location of the assembly 101. Referring to FIGS. 1, 8, and 12, the server computing device 310 receives the data at block 1202. That is, the data that is transmitted by the receiver portion of the locating system 103 (e.g., the first component 105 or the second component 107) that is transmitted via the lift communications network 302 is received by the server computing device 310. As described herein, the data may generally include the code that corresponds to the location of the receiver (and thus the location of the assembly 101), a time stamp indicating the time at which the code was received, an identity of the receiver and/or the assembly, and/or the like.

In order to determine information encoded by the code (e.g., determine a location that corresponds to the code), the server computing device 310 searches a database for the code at block 1204. For example, the server computing device may access a look-up table or the like that corresponds individual codes to particular locations and/or identities of the components transmitting the codes. That is, in embodiments where the transmitter is the second component 107 of the locating system 103 that is at a fixed location on or adjacent to the rail 102, the code may correspond to the fixed location of the second component 107, and the location may be stored in the database. In embodiments where the transmitter is the first component 105 of the locating system 103 located on the moving assembly 101, the code may correspond to an identity of the assembly 101, and the additional data that is received from the receiver (e.g., the second component 107) is indicative of the fixed location of the receiver, which is stored in the database. The server computing device 310 determines at block 1206 whether the code is contained within the database. That is, the server computing device 310 may determine at block 1206 whether a code exists in the database that corresponds to the code received in the data. If a code does not exist in the database, the process may continue to block 1208. If a code exists in the database, the process may continue to block 1210.

At block 1208, an error message is logged by the server computing device 310. That is, the server computing device 310 may generate or amend a log file that indicates that a code corresponding to the code received in the data was not found in the database. In some embodiments, the log file may be later used to correct code errors, to transmit an error message to a user (e.g., via the hand control unit 112 and/or the user computing device 320, and/or the like).

At block 1210, the location and identity of the assembly 101 is determined from the code based on the database entry. In embodiments where the receiver portion of the locating system 103 is the first component 105 located on the assembly 101, the database entry for the location may correspond to the fixed position at which the transmitter (e.g., the second component 107 of the locating system 103) is located, and the database entry for the identity of the assembly 101 may correspond to the additional data transmitted by the receiver. In embodiments where the receiver portion of the locating system 103 is the second component 107 located on or near the rail 102 in a fixed position, the database entry for the location may correspond to the fixed position at which the receiver (e.g., the second component 107 of the locating system 103) is located, and the database entry for the identity of the assembly may correspond to the code that was transmitted by the transmitter (e.g., the first component 105 of the locating system 103).

As previously described herein, certain spaces may have sub-locations thereof. As such, an additional determination of a subspace may be completed to accurately determine the location of the assembly 101. Accordingly, a determination is made at block 1212 as to whether multiple sub-locations exist. This may be completed, for example, by parsing the data in the database for annotations or the like that are indicative of a sub-location. For example, a location entry in a look-up table may include an annotation that multiple sub-locations exist. In another example, the code stored in the database may correspond to a plurality of entries, where each entry is a sub-location. Thus, if the processes described with respect to blocks 1206 and 1210 result in a plurality of entries matching the code, then the determination at block 1212 may be that multiple sub-locations exist. If multiple sub-locations do not exist, the process may move to block 1214. If multiple sub-locations exist, the process may move to block 1216.

At block 1214, the location and the identity of the assembly 101, as well as the time at which the location was sensed, is logged by the server computing device 310. That is, the server computing device 310 may generate or update a log file with the location and identity of the assembly 101 such that the log file can be accessed to determine the location of the assembly 101, as well as identification of the assembly (e.g., a serial number or the like) at any time. In addition to the location and identity of the assembly, the time at which the location was sensed is also recorded in the log file. Accordingly, when the log file is accessed in the future (e.g., by a user of the user computing device 320, by the server computing device 310, and/or by another external device), a complete record of which assembly 101 was located at a particular location at a particular time is available. As a result, the location of the assembly 101 can be accurately and automatically tracked as the assembly 101 moves around a space (e.g., a hospital or other medical facility) without any human intervention in tracking the location thereof. In some embodiments, the location and identification of the assembly 101, as well as the subject and/or user associated with the assembly 101, may be displayed on an external device, such as a monitor or other display device coupled to the user computing device 320 (e.g., a display at a nurse's station or the like).

Referring now to FIGS. 1, 8-9, and 12, a sub-location is determined by the server computing device 310 in embodiments where a plurality of sub-locations exist at a particular location at block 1216. Such a determination may be completed by receiving additional data or further analyzing the data that has been received from the assembly 101. For example, in embodiments where a plurality of second components (e.g., second component 107b and second component 107c) are located in respective subspaces 404 and 406 respectively, a determination of the subspace may include receiving data corresponding to a code from that second component (e.g., second component 107b or second component 107c), searching the database for the code, and identifying a location/identity similar to the processes described hereinabove with respect to blocks 1202-1210. In another example, in embodiments where additional data corresponding to an input received via the hand control unit 112, the additional data may be used to determine a sub-location. For example, if the rail 102 extends into a shared room and splits into sub-segments corresponding to areas where each subject is located within the room (e.g., a bed area for each subject in the room) and a database has a record of which subject is in each area of the room, then information inputted into the hand control unit 112 (e.g., scanning a barcode 126 on an identification bracelet 124 of a subject S (FIG. 5), receiving user inputs corresponding to an identification of a subject, and/or the like) may be used to correlate the subject identity with the sub-location.

At block 1218, the sub-location and the identity of the assembly 101, as well as the time at which the sub-location was sensed, is logged by the server computing device 310. That is, the server computing device 310 may generate or update a log file with the sub-location and identity of the assembly 101 such that the log file can be accessed to determine the sub-location of the assembly 101, as well as identification of the assembly (e.g., a serial number or the like) at any time. In addition to the sub-location and identity of the assembly, the time at which the sub-location was sensed is also recorded in the log file. Accordingly, when the log file is accessed in the future (e.g., by a user of the user computing device 320, by the server computing device 310, and/or by another external device), a complete record of which assembly 101 was located at a particular sub-location at a particular time is available. As a result, the sub-location of the assembly 101 can be accurately and automatically tracked as the assembly 101 moves around a space (e.g., a hospital or other medical facility) without any human intervention in tracking the location thereof. In some embodiments, the sub-location and identification of the assembly 101, as well as the subject and/or user associated with the assembly 101, may be displayed on an external device, such as a monitor or other display device coupled to the user computing device 320 (e.g., a display at a nurse's station or the like).

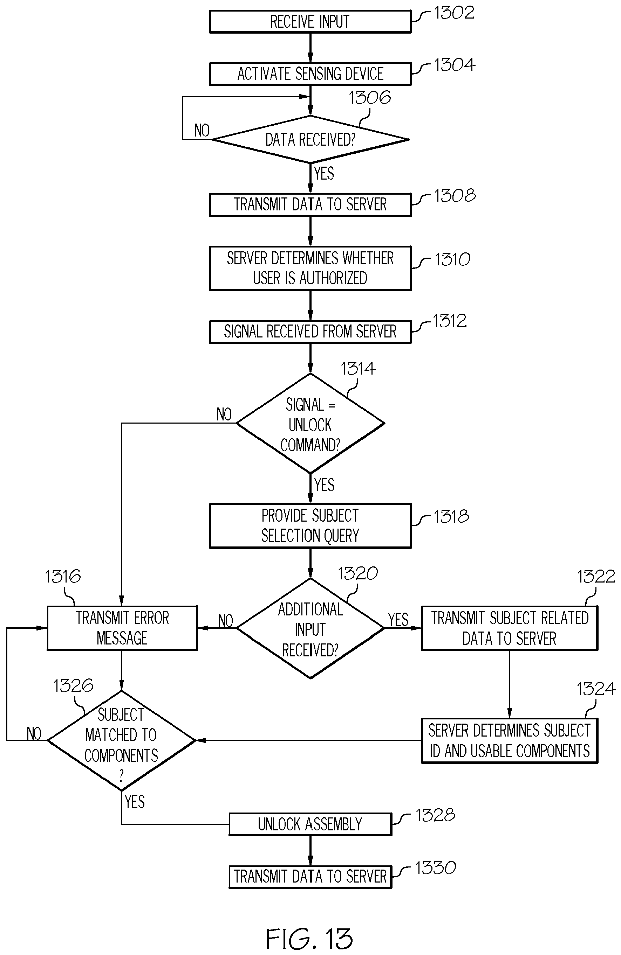

As noted hereinabove, additional data or information may be received by the server computing device 310, which may be used to complete various processes other than determining a location and identity of the assembly 101. For example, FIG. 13 depicts a flow diagram of an illustrative method of pairing a user and a subject to the assembly 101. Referring to FIGS. 1, 7A, 8, and 13, an input is received by the assembly at block 1302. That is, the assembly 101 or a component thereof (e.g., the hand control unit 112) may receive an input from a user. The input may generally be indicative of a use of the assembly 101 or a portion thereof or an intent to use the assembly 101 or a portion thereof. For example, the user may press a button on the hand control unit 112 (e.g., to power on the assembly, to awaken the assembly from a sleep mode, or the like). In some embodiments, movement of the assembly 101 along the rail 102 may cause an actuation of the assembly that functions in a manner similar to the input. That is, if the assembly 101 includes an inertial power generator or the like that generates electrical power as a result of movement of the assembly 101 along the rail 102, such movement may cause the inertial power generator to actuate. Accordingly, the actuation may be recognized as an input indicative of a use of the assembly 101.

At block 1304, the sensing device 117 is activated. That is, the sensing device 117 may be powered on or otherwise rendered active for the purposes of sensing. For example, in embodiments where the sensing device 117 is an imaging device, the sensing device 117 may be actuated such that it images an area for a barcode or the like. Such an actuation may be in response to a particular input provided by the user that is received at block 1302. For example, in embodiments where the hand control unit 112 contains a trigger button or the like that is used to direct the sensing device 117, the sensing device may be activated at block 1304 when the user presses the trigger button to provide the input at block 1302.

A determination is made at block 1306 as to whether data has been received. That is, a determination is made by the processing device 210 as to whether the sensing device 117 senses a transmission, code, or the like that contains data. For example, in embodiments where the sensing device 117 is an imaging device, the determination at block 1306 may be that data is received when a barcode or the like is obtained from the image data received by the sensing device 117. The barcode or the like may be a barcode on the user's ID badge, where the barcode includes encoded information that is used to identify and/or authenticate the user in some embodiments. In another example, in embodiments where the sensing device 117 is an imaging device, the determination at block 1306 may be that data is received when the user's fingerprint is scanned by the sensing device 117. If no data is received, the sensing device 117 may continue to sense until data is received, and thus the process repeats at block 1306. If data is received, the process may proceed to block 1308.

At block 1308, the data that is received by the sensing device 117 is transmitted to the server computing device 310. That is, the network interface hardware 240 of the assembly 101 may transmit the data via the lift communications network 302 to the server computing device 310.

Still referring to FIGS. 1, 8, and 13, the server computing device 310 determines whether the user is authorized to use the assembly 101 at block 1310. That is, the server computing device 310 obtains the data, determines information from the data (e.g., the user's name, a code corresponding to the user's identity, an identity of the assembly 101 and/or the components or accessories attached thereto, or the like), searches an authorization database for the user and/or the assembly 101 (as well as components or accessories attached thereto), determines from the authorization database whether the user is authorized to use the assembly and/or the components or accessories attached thereto, and generates an "authorized" signal or an "unauthorized" signal as a result. The "authorized" signal may generally include a command to unlock the assembly 101 such that a user can operate the assembly 101 via the hand control unit 112 once a subject is confirmed, as descried herein. The "unauthorized" signal may not include such a command to unlock the assembly 101. In addition to generating the signal, the server computing device 310 may generate or update a log to indicate information pertaining to the user's request to unlock the assembly 101 and the response provided. The "authorized" signal or the "unauthorized" signal are then transmitted back to the assembly 101 such that the signal is received at block 1312. That is, the signal is transmitted by the server computing device 310 via the lift communications network 302 to the network interface hardware 240 of the assembly 101.

Referring to FIGS. 1, 7A, 8, and 13, a determination is made by the assembly 101 at block 1314 as to whether the received signal includes an unlock command. For example, the processing device 210 of the assembly 101 may determine whether the signal is the "authorized" signal containing the unlock command or the "unauthorized" signal not containing the unlock command. If the processing device 210 of the assembly 101 determines that the signal is the "unauthorized" signal that does not contain the unlock command, the process may proceed to block 1316. If the processing device 210 of the assembly 101 determines that the signal is the "authorized" signal that contains the unlock command, the process may proceed to block 1318.

At block 1316, an error message is transmitted to the user. That is, the processing device 210 of the assembly 101 may direct one or more components of the assembly 101 to provide a notification to the user that the user is not authorized to use the assembly. For example, the processing device 210 may direct the display 114 of the hand control unit 112 to display a notification, such as "UNAUTHORIZED USER" or the like. In another example, the processing device 210 may cause a speaker or the like to emit a tone and/or a recorded message that indicates that the user is unauthorized. In addition, the assembly 101 may remain locked for use such that the user cannot operate the hand control unit 112 to move the lifting strap 108 up or down, change settings, and the like. Other error messages that may be transmitted to the user at block 1316 include, but are not limited to, an indication that no additional input has been received regarding an identity of a subject, an indication that the assembly 101 cannot be used for a particular subject, and/or the like.

At block 1318, a subject selection query is presented to the user. That is, the processing device 210 of the assembly 101 may direct one or more components of the assembly 101 to request that the user select or otherwise identify the subject to be hoisted using the assembly 101. For example, the display 114 of the hand control unit 112 may be directed by the processing device 210 to display a graphic, text, or the like requesting that the user select a subject. In addition, the processing device 210 may provide a list of potential subjects, which may be based on the determined location of the assembly 101, as described herein. For example, if the assembly 101 has been determined to be in a particular space such as a room containing 2 subjects, the list of potential subjects may include only the two subjects that are assigned to the room.

Once the query is provided to the user according to block 1318, the user may select a subject. In some embodiments, the user may use the user interface controls 116 of the hand control unit 112 to highlight and select the correct user from the list provided. In other embodiments, the user may scan a barcode or the like (using the sensing device 117 of the hand control unit 112) that is indicative of the subject's identity (e.g., the barcode 126 located on an identification bracelet 124 as shown in FIG. 5). In yet other embodiments, the user may voice a code, the subject's name, or the like, which is received by a microphone or the like.

Accordingly, a determination is made at block 1320 as to whether the input mentioned above has been received from the user. If the input has not been received, the process may proceed to block 1316. Alternatively, the process may repeat at block 1320 such that the process does not continue until an input has been received. If an input has not been received within a particular period of time, the process may time out and then proceed to block 1316. If the input has been received, the process may proceed to block 1322.

At block 1322, subject related data is transmitted to the server computing device 310. That is, data corresponding to the information inputted by the user is transmitted by the network interface hardware 240 of the assembly 101 via the lift communications network 302 to the server computing device 310. Upon receiving the data, the server computing device 310 determines the identification of the subject and whether the assembly 101 and/or various components or attached thereto are suitable for the subject. For example, the server computing device 310 may determine the subject identity and access electronic medical records or the like pertaining to the subject to ensure that the subject's injuries, physical characteristics, and/or the like are suitable for the assembly 101 and/or the components or accessories attached thereto. In one illustrative example, the server computing device 310 may determine that the subject has a mass of 150 kg and may verify that the assembly 101 and/or the components or accessories attached thereto can support such a mass. As such, a determination is made at block 1326 as to whether the subject is matched to the components or accessories of the assembly 101. If so, the process may proceed to block 1328. If not, the process may proceed to block 1316.

At block 1328, the assembly may be unlocked for use with the selected subject. That is, the various components of the hand control unit 112 may be unlocked for a user to operate the hand control unit 112 to cause the various components of the assembly 101 to function to lift the subject (e.g., cause the lifting strap 108 to be moved up and down). In addition, other data that may be generated as a result of use of the assembly 101 may be transmitted to the server computing device 310 at block 1330 such that the server computing device 310 can log the data for future retrieval.

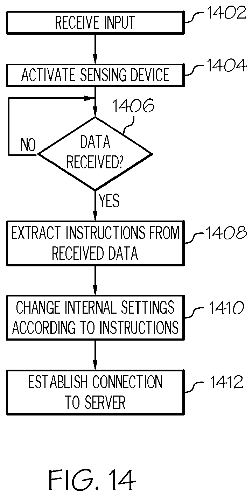

Before an assembly 101 can be used for the purposes of tracking a location thereof 101, authenticating a user, and/or verifying that a subject can be used with the assembly 101 as described herein, it may be necessary to register the assembly on the lift communications network 302 such that the assembly 101 can communicate with the server computing device 310. Accordingly, FIG. 14 depicts a flow diagram of an illustrative method of establishing communications between the assembly 101 and the server computing device 310.

Referring to FIGS. 1, 7A, 8, and 14, an input is received at block 1402. That is, the assembly 101 or a component thereof (e.g., the hand control unit 112) may receive an input from a user. The input may generally correspond to a command to turn on or otherwise activate the assembly 101 for pairing.

At block 1404, the sensing device 117 is activated. That is, the sensing device 117 may be powered on or otherwise rendered active for the purposes of sensing. For example, in embodiments where the sensing device 117 is an imaging device, the sensing device 117 may be actuated such that it images an area for a barcode or the like. Such an actuation may be in response to a particular input provided by the user that is received at block 1402. For example, in embodiments where the hand control unit 112 contains a trigger button or the like that is used to direct the sensing device 117, the sensing device may be activated at block 1404 when the user presses the trigger button to provide the input at block 1402.

A determination is made at block 1406 as to whether data has been received. That is, a determination is made by the processing device 210 as to whether the sensing device 117 senses a transmission, code, or the like that contains data. For example, in embodiments where the sensing device 117 is an imaging device, the determination at block 1406 may be that data is received when a barcode or the like is obtained from the image data received by the sensing device 117. The barcode or the like may be a barcode on a configuration sheet or the like, where the barcode includes encoded information that is used to configure the assembly for communicating on the network, such as a service set identifier (SSID) of a network, a password for accessing the network, communication port settings, and/or the like. If no data is received, the sensing device 117 may continue to sense until data is received, and thus the process repeats at block 1406. If data is received, the process may proceed to block 1408.

At block 1408, instructions in the received data are extracted by the processing device 210 of the assembly 101. As mentioned above, the instructions may include configuration instructions for configuring the assembly 101 to be able to communicate with the lift communications network 302 and/or register the assembly 101 with the communications network. That is, the instructions may include details regarding the SSID of the network, a password for accessing the network, communications port settings, and/or the like. The processing device 210 then changes the various internal settings to correspond to the information that was received and extracted at block 1410. That is, the processing device 210 may cause the memory 220 to update the operating logic 222 and/or cause the data storage device 250 to update the configuration data 256 in accordance with the received instructions.

At block 1412, a connection to the server computing device 310 is established. That is, the assembly 101 connects to the lift communications network 302 using the information received and extracted at block 1408 such that the network interface hardware 240 can communicate with the server computing device 310 via the lift communications network 302.

In the embodiments described herein and shown in the figures, the rail-mounted lift system 100 is constructed with the assembly 101, the locating system 103 (including the first component 105 and the second component thereof 107), and the server computing device 310 such that the location of the lift assembly can be determined at particular points along the length of the rail 102 (e.g., points where the second component 107 is attached). However, it is also contemplated that certain components of the rail-mounted lift system 100 may be separately provided as a kit of parts which may be used to retro fit lift systems which do not have location tracking capabilities. For example, in one embodiment, a kit of parts for retrofitting a rail-mounted lift system for tracking a location of a lift unit along a length of a rail may include at least one first component and at least one second component (e.g., at least one receiver and at least one transmitter) which are packaged together. In addition, the kit of parts for retrofitting a rail-mounted lift system may also include network interface hardware coupled to the first component and/or the second component, as well as a server computing device communicatively coupled to the network interface hardware. The kit may further include instructions for affixing the one or more first components and the one or more second components to existing components of a rail-mounted lift system as described herein.

As noted above, the kit of parts may be used to retrofit and convert an existing rail-mounted lift system to a rail-mounted lift system with location tracking capabilities. The various parts of the kit of parts may be installed on or near the rail and on or in the assembly of the existing rail-mounted lift system as described hereinabove, thereby converting the existing rail-mounted lift system to a rail-mounted lift system having a location tracking system.

Based on the foregoing, it should now be understood that the embodiments shown and described herein relate to a rail-mounted lift system for lifting and supporting subjects. The rail-mounted lift system includes an assembly including at least a carriage, a lift unit, and a hand control unit, which are slidably coupled via the carriage to a rail. The rail-mounted lift system further includes a locating system that includes a first component mounted on the lift unit and a second component mounted on or near the rail. In addition, various components of the assembly and the locating system are communicatively coupled to a server computing device. A code provided by one of the first component or the second component is detected by the other of the first component and the second component as the carriage moves the assembly along the rail and moves the first component and second component in proximity to each other. The code is transmitted to the server computing device, which uses the code to determine the location of the assembly. In addition, the hand control unit can be used in conjunction with the server computing device to verify that a user of the assembly is authorized to use the assembly, and to verify that a subject can be used with the assembly.

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

While particular embodiments have been illustrated and described herein, it should be understood that various other changes and modifications may be made.

Embodiments of the invention can be described with reference to the following numbered clauses, with preferred features laid out in the dependent clauses:
1. A rail-mounted lift system comprising:
   a rail comprising a carriage support channel formed in the rail;
   a carriage slidably disposed in the carriage support channel of the rail for relative movement to the rail;
   a lift unit coupled to the carriage, the lift unit comprising a motor that extends and retracts a lifting strap; and
   a locating system comprising a first component and a second component that is separate from the first component, wherein one of the first component and the second component is a transmitter providing a code that corresponds to a predetermined location and another one of the first component and the second component is a receiver detecting the code when the first component and the second component are moved in proximity to one another due to movement of the carriage along the rail.
2. The rail-mounted lift system of clause 1, further comprising:
   network interface hardware communicatively coupled to the locating system, the network interface hardware receiving the code from the receiver of the locating system and transmitting data comprising the code to a remote server.
3. The rail-mounted lift system of clause 1 or clause 2, further comprising:
   a hand control unit communicatively coupled to the lift unit, the hand control unit comprising:
   a display;
   one or more user interface controls; and
   a sensing device.
4. The rail-mounted lift system of clause 3, wherein the sensing device is an imaging device.
5. The rail-mounted lift system of any one of clauses 1-4, wherein the first component is coupled to the carriage or the lift unit and the second component is positioned on or near the rail.
6. The rail-mounted lift system of any one of clauses 1-5, wherein:
   the transmitter is a barcode, a QR code, an infrared (IR) beacon, a radio frequency identification (RFID) emitter or tag, or a wireless transmitter; and
   the receiver is an imaging device, an IR receiver, an RFID detector, or a wireless receiver.
7. The rail-mounted lift system of any one of clauses 1-6, wherein the first component is the receiver and the second component is the transmitter.
8. The rail-mounted lift system of any one of clauses 1-6, wherein the first component is the transmitter and the second component is the receiver.
9. The rail-mounted lift system of clause 8, wherein the second component is communicatively coupled to network interface hardware that receives the code from the second component and transmits data comprising the code to a server computing device remotely located from the network interface hardware.
10. The rail mounted lift system of any one of clauses 1-9, wherein the predetermined location is a space or a defined subspace within the space.
11. The rail-mounted lift system of any one of clauses 1-9, wherein:
   the rail further comprises an upper portion, a first support flange, and a second support flange that define the carriage support channel; and
   the carriage comprises:
      a carriage body, and
      at least one pair of support wheels rotatably coupled to the carriage body, the at least one paid of support wheels slidably engaged with the support flange and the second support flange.
12. The rail-mounted lift system of clause 11, wherein:
   the carriage body comprises a receiving slot; and
   the lift unit comprises a connection rail, wherein the connection rail of the lift unit is slidably received in the receiving slot to couple the lift unit to the carriage body.
13. The rail-mounted lift system of clause 11 or clause 12, wherein the rail further comprises a first sidewall integrally formed with the upper portion, and a second sidewall integrally formed with the upper portion and opposed to the first sidewall, the first sidewall, the second sidewall and the upper portion defining the carriage support channel, wherein:
   the first support flange extends into the carriage support channel from the first sidewall; and
   the second support flange extends into the carriage support channel from the second sidewall.
14. The rail-mounted lift system of any one of clauses 1-13, wherein the carriage comprises a first end bumper attached to a first end of the carriage and a second end bumper attached to a second end of the carriage.
15. The rail-mounted lift system of any one of clauses 1-14, wherein the lift unit and at least one of the first component and the second component of the locating system are electrically coupled to a power source.
16. The rail-mounted lift system of clause 15, wherein the power source is a battery.
17. A system comprising:
   a server computing device; and
   a rail-mounted lift system comprising:
      a rail comprising a carriage support channel formed in the rail,
      at least one assembly comprising a carriage slidably disposed in the carriage support channel of the rail for relative movement to the rail, the at least one assembly communicatively coupled to the server computing device, and
      a locating system comprising a first component and a second component that is separate from the first component, wherein one of the first component and the second component is a transmitter providing a code that corresponds to a predetermined location and another one of the first component and the second component is a receiver detecting the code when the first component and the second component are moved in proximity to one another due to movement of the carriage along the rail.
18. The system of clause 17, wherein the at least one assembly further comprises network interface hardware that transmits data comprising the code and an identification of the at least one assembly to the server computing device.
19. The system of clause 18, wherein the server computing device determines the location of the at least one assembly based on the data received from the network interface hardware.
20. The system of any one of clauses 17-19, wherein the at least one assembly further comprises:
   a lift unit coupled to the carriage; and
   a hand control unit communicatively coupled to the lift unit, the hand control unit comprising:
      a display;
      one or more user interface controls; and
      a sensing device,
   wherein data relating to at least one of a subject and a user is generated based on one or more inputs received from at least one of the one or more user interface controls and the sensing device.
21. The system of clause 20, wherein the at least one assembly further comprises network interface hardware communicatively coupled to the hand control unit, the network interface hardware transmitting the data to the server computing device.
22. The system of clause 21, wherein the server computing device determines at least one of an identity of the subject and an identity of the user from the data.
23. The system of clause 21 or clause 22, wherein the server computing device authenticates the user to operate the at least one assembly.
24. The system of any one of clauses 21-23, wherein the server computing device determines that the subject is matched to one or more components of the assembly.
25. The system of any one of clauses 21-24, wherein a barcode that is readable by the sensing device provides one or more programming instructions for communicatively coupling the network interface hardware to the server computing device.
26. The system of any one of clauses 17-25, wherein:
   the at least one assembly comprises a plurality of assemblies, each one of the plurality of assemblies is communicatively coupled to the server computing device; and
   the locating system comprises a plurality of first components, each one of the plurality of first components coupled to each one of the plurality of assemblies.
27. A kit of parts for retrofitting a rail-mounted lift system for automatically determining a location of an assembly along a length of a rail, the kit of parts comprising:
   a locating system comprising a first component and a second component, wherein one of the first component and the second component is a transmitter that provides a code corresponding to a predetermined location and another one of the first component and the second component is a receiver;
   network interface hardware communicatively coupled to the receiver;
   a server computing device communicatively coupled to the network interface hardware; and
   instructions for coupling the first component to a carriage or a lift unit of the rail-mounted lift system, coupling the second component on or near a rail of the rail-mounted lift system, and positioning the first component and the second component such that, when the first component and the second component are moved in proximity to one another due to movement of the carriage along the rail, a code is transmitted between the first component and the second component.

## Claims

1. A rail-mounted lift system comprising:
a rail comprising a carriage support channel formed in the rail;
a carriage slidably disposed in the carriage support channel of the rail for relative movement to the rail;
a lift unit coupled to the carriage, the lift unit comprising a motor that extends and retracts a lifting strap; and
a locating system comprising a first component and a second component that is separate from the first component, wherein one of the first component and the second component is a transmitter providing a code that corresponds to a predetermined location and another one of the first component and the second component is a receiver detecting the code when the first component and the second component are moved in proximity to one another due to movement of the carriage along the rail.

2. The rail-mounted lift system of claim 1, further comprising:
a hand control unit communicatively coupled to the lift unit, the hand control unit comprising:
a display;
one or more user interface controls; and
a sensing device.

3. The rail-mounted lift system of any one of claims 1-2, wherein the first component is coupled to the carriage or the lift unit and the second component is positioned on or near the rail.

4. The rail-mounted lift system of any one of claims 1-3, wherein:
the transmitter is a barcode, a QR code, an infrared (IR) beacon, a radio frequency identification (RFID) emitter or tag, or a wireless transmitter; and
the receiver is an imaging device, an IR receiver, an RFID detector, or a wireless receiver.

5. The rail-mounted lift system of any one of claims 1-4, wherein the first component is the receiver and the second component is the transmitter.

6. The rail-mounted lift system of any one of claims 1-4, wherein the first component is the transmitter and the second component is the receiver, wherein the second component is communicatively coupled to network interface hardware that receives the code from the second component and transmits data comprising the code to a server computing device remotely located from the network interface hardware.

7. The rail-mounted lift system of any one of claims 1-6, wherein:
the rail further comprises an upper portion, a first support flange, and a second support flange that define the carriage support channel; and
the carriage comprises:
a carriage body, and
at least one pair of support wheels rotatably coupled to the carriage body, the at least one paid of support wheels slidably engaged with the support flange and the second support flange.

8. The rail-mounted lift system of claim 7, wherein:
the carriage body comprises a receiving slot; and
the lift unit comprises a connection rail, wherein the connection rail of the lift unit is slidably received in the receiving slot to couple the lift unit to the carriage body.

9. The rail-mounted lift system of claim 7 or claim 8, wherein the rail further comprises a first sidewall integrally formed with the upper portion, and a second sidewall integrally formed with the upper portion and opposed to the first sidewall, the first sidewall, the second sidewall and the upper portion defining the carriage support channel, wherein:
the first support flange extends into the carriage support channel from the first sidewall; and
the second support flange extends into the carriage support channel from the second sidewall.

10. The rail-mounted lift system of any one of claims 1-9, wherein the carriage comprises a first end bumper attached to a first end of the carriage and a second end bumper attached to a second end of the carriage.

11. The rail-mounted lift system of any one of claims 1-10, wherein the lift unit and at least one of the first component and the second component of the locating system are electrically coupled to a power source, preferably a battery.

12. A system comprising:
a server computing device; and
the rail-mounted lift system of any one of claims 1-11.

13. The system of claim 12, wherein the at least one assembly further comprises network interface hardware that transmits data comprising the code and an identification of the at least one assembly to the server computing device, wherein the server computing device determines the location of the at least one assembly based on the data received from the network interface hardware.

14. The system of claim 12 or claim 13, wherein the at least one assembly further comprises network interface hardware communicatively coupled to the hand control unit, the network interface hardware transmitting the data to the server computing device, wherein the server computing device determines at least one of an identity of the subject and an identity of the user from the data, authenticates the user to operate the at least one assembly, and/or determines that the subject is matched to one or more components of the assembly.

15. A kit of parts for retrofitting a rail-mounted lift system for automatically determining a location of an assembly along a length of a rail, the kit of parts comprising:
a locating system comprising a first component and a second component, wherein one of the first component and the second component is a transmitter that provides a code corresponding to a predetermined location and another one of the first component and the second component is a receiver;
network interface hardware communicatively coupled to the receiver;
a server computing device communicatively coupled to the network interface hardware; and
instructions for coupling the first component to a carriage or a lift unit of the rail-mounted lift system, coupling the second component on or near a rail of the rail-mounted lift system, and positioning the first component and the second component such that, when the first component and the second component are moved in proximity to one another due to movement of the carriage along the rail, a code is transmitted between the first component and the second component.
